**Europäisches Patentamt**

**European Patent Office**

**Office européen des brevets**

(19)

(11) Veröffentlichungsnummer: **0 027 658**
**B1**

# EUROPÄISCHE PATENTSCHRIFT

(12)

(45) Veröffentlichungstag der Patentschrift:
**26.05.82**

(51) Int. Cl.³: **C 07 C 87/60,** C 07 C 85/00

(21) Anmeldenummer: **80106381.9**

(22) Anmeldetag: **20.10.80**

(54) **Verfahren zur Herstellung von 3-Nitro-4-aminotoluol.**

(30) Priorität: **23.10.79 DE 2942676**

(43) Veröffentlichungstag der Anmeldung:
**29.04.81 Patentblatt 81/17**

(45) Bekanntmachung des Hinweises auf die Patenterteilung:
**26.05.82 Patentblatt 82/21**

(84) Benannte Vertragsstaaten:
**BE CH DE FR GB LI NL SE**

(56) Entgegenhaltungen:
**DE-C- 767 072**
**GB-A- 469 080**
**US-A-2 686 810**

(73) Patentinhaber: **BAYER AG, Zentralbereich Patente, Marken und Lizenzen, D-5090 Leverkusen 1, Bayerwerk (DE)**

(72) Erfinder: **Schössler, Willi, Dr., Hahnenweg 2, D-5000 Köln 80 (DE)**
Erfinder: **Blank, Heinz Ulrich, Dr., Am Geusfelde 35, D-5068 Odenthal (DE)**

Verfahren zur Herstellung von 3-Nitro-4-aminotoluol

Die vorliegende Erfindung betrifft ein Verfahren zur Herstellung von 3-Nitro-4-aminotoluol aus p-Acetylaminotoluol durch Nitrierung in Gegenwart von inerten, mit Wasser nicht mischbaren organischen Lösungsmitteln.

Es ist bekannt, 3-Nitro-4-aminotoluol durch Nitrieren von 4-Acetylaminotoluol mit Hilfe eines Säuregemisches aus Salpetersäure und Schwefelsäure in einem inerten Lösungsmittel, wie Methylenchlorid, herzustellen (DE-AS 2 226 405).

Dieses Verfahren hat jedoch den Nachteil, dass neben dem gewünschten 3-Nitro-4-acetylaminotoluol noch das 2-Nitro-4-acetylaminotoluol als Nebenprodukt entsteht, was nach der alkalischen Entacetylierung die Qualität des 3-Nitro-4-aminotoluols beeinträchtigt.

Weiterhin sind die Ausbeuten an gewünschtem Reaktionsprodukt mit 88–91% der Theorie für ein wirtschaftliches Verfahren nicht befriedigend.

Ferner ist bekannt, 3-Nitro-4-acetylaminotoluol herzustellen, indem in einem Eintopfverfahren, ausgehend von p-Aminotoluol, dieses zuerst mit Acetanhydrid in Gegenwart von Monochlorbenzol bei erhöhter Temperatur acetyliert und anschliessend mit 80%iger Salpetersäure bei 50°C nitriert wird (vgl. US-PS 2 459 002, Beispiel 4).

Nachteilig bei diesem Verfahren sind die geringen Ausbeuten und die schlechten Qualitäten an 3-Nitro-4-aminotoluol, wie eine Nacharbeitung des Beispiels 4 der US-PS zeigt (in Beispiel 4 der US-PS fehlt nämlich eine Angabe zur Ausbeute und Produktqualität).

Da bei dem Verfahren der US-PS 2 459 002 die Acylierung und Nitrierung nacheinander, d.h. ohne Isolierung des acylierten Zwischenproduktes durchgeführt werden, besteht das Nitriergemisch immer aus überschüssigem Acylierungsmittel, freigesetzter organischer oder anorganischer Säure und Salpetersäure. Dies hat jedoch den Nachteil, dass neben den geringen Ausbeuten und der schlechten Qualitäten an 3-Nitro-4-aminotoluol bei der anschliessenden Neutralisation des Reaktionsgemisches mehr Alkali benötigt wird als eigentlich für die Neutralisation der Salpetersäure erforderlich ist. Durch die Neutralisation geht das überschüssige Acylierungsmittel und die freigesetzte organische oder anorganische Säure für eine weitere wirtschaftliche Nutzung verloren und zusätzlich wird das Abwasser durch die bei der Neutralisation gebildeten Salze belastet.

Weiterhin wurde bei der Nacharbeitung des Beispiels 4 der US-PS beobachtet, dass, wenn das 3-Nitro-4-acetylaminotoluol ohne Zwischenisolierung in saurem Medium gespalten wird, zum Teil Verharzung eintritt. Zur Isolierung des 3-Nitro-4-aminotoluols ist es daher erforderlich, die Reaktionslösung zuvor mit Aktivkohle zu behandeln.

Es wurde nun ein Verfahren zur Herstellung von 3-Nitro-4-aminotoluol durch Nitrieren von p-Acetylaminotoluol in Gegenwart von inerten, mit Wasser nicht mischbaren organischen Lösungsmitteln bei Temperaturen im Bereich von 0 bis 80°C und anschliessender alkalischer Verseifung und Aufarbeitung gefunden, das dadurch gekennzeichnet ist, dass man die Nitrierung mit 60 bis 100 gew.-%iger Salpetersäure in Gegenwart von Methylenchlorid durchführt.

Bevorzugt wird bei dem erfindungsgemässen Verfahren eine Salpetersäure mit einer Konzentration von 85 bis 100 Gew.-% eingesetzt, wobei die Nitrierung mit 98%iger Salpetersäure besonders bevorzugt ist.

Nach dem erfindungsgemässen Verfahren werden pro Mol p-Acetylaminotoluol mindestens 2 Mol 100 gew.-%ige Salpetersäure, bevorzugt 2 bis 6 Mol, besonders bevorzugt 2,2 bis 4,5 Mol, 100 gew.-%ige Salpetersäure eingesetzt.

Bei Verwendung von 60 gew.-%iger Salpetersäure werden mindestens 6 Mol Salpetersäure pro Mol p-Acetylaminotoluol eingesetzt. Bevorzugt werden pro Mol p-Acetylaminotoluol 6 bis 12 Mol, besonders bevorzugt 6,5 bis 8 Mol, 60 gew.-%ige Salpetersäure eingesetzt.

Für Salpetersäure-Konzentrationen, die zwischen 60 und 100 Ge.-% liegen, wird zweckmässigerweise das günstigste Molverhältnis von Salpetersäure zu p-Acetylaminotoluol durch Vorversuche ermittelt.

Beispielsweise beträgt das Molverhältnis von 98 gew.-%iger Salpetersäure, die bevorzugt in das erfindungsgemässe Verfahren eingesetzt wird, zu p-Acetylaminotoluol 2,2 bis 6,5:1, bevorzugt 2,4 bis 5:1.

Die Menge an Methylenchlorid ist nicht kritisch und kann in weiten Bereichen variieren. Im allgemeinen beträgt die Menge an Methylenchlorid etwa 400 bis etwa 2000 ml pro Mol p-Acetylaminotoluol. Bevorzugt werden auf 1 Mol p-Acetylaminotoluol 450 bis 800 ml, besonders bevorzugt 500 bis 700 ml Methylenchlorid eingesetzt.

Als Einsatzprodukte können nach dem erfindungsgemässen Verfahren sowohl technisches p-Acetylaminotoluol mit einem Gehalt von etwa 96 bis 99,5 Gew.-% als auch reines p-Acetylaminotoluol mit einem Gehalt von $\geq$ 99,5 Gew.-% eingesetzt werden. Technisches p-Acetylaminotoluol kann, bevor es umgesetzt wird, zur Reinigung beispielsweise mit Methylenchlorid gewaschen oder umkristallisiert werden.

Die erfindungsgemässe Nitrierung wird im allgemeinen bei Temperaturen im Bereich von etwa 0 bis etwa 80°C, bevorzugt bei 20 bis 50°C, besonders bevorzugt bei 30 bis 40°C, durchgeführt.

Die Reaktionszeit der Nitrierung hängt im wesentlichen von der Salpetersäurekonzentration und der Menge der eingesetzten Salpetersäure ab. Die Reaktionszeit ist um so kürzer, je höher konzentriert die Salpetersäure und je grösser der Überschuss an Salpetersäure, bezogen auf das Ausgangsprodukt, ist. Im allgemeinen betragen die Reaktionszeiten etwa 15 Minuten bis etwa 3 Stunden.

Das erfindungsgemässe Verfahren kann bei Normaldruck als auch bei vermindertem oder erhöhtem Druck, beispielsweise bei Drücken im Bereich von etwa 0,3 bis etwa 3,5 bar, bevorzugt bei etwa 0,4 bis etwa 1,4 bar, besonders bevorzugt bei 0,6 bis 1 bar, durchgeführt werden.

Neben der diskontinuierlichen Verfahrensweise ist auch eine kontinuierliche Durchführung des erfindungsgemässen Verfahrens möglich.

Das erfindungsgemässe Verfahren kann in der Weise durchgeführt werden, dass man das Einsatzprodukt gelöst oder suspendiert in Methylenchlorid vorlegt und mit Salpetersäure im zuvor beschriebenen Temperaturbereich versetzt. Dabei ist es im allgemeinen nicht erforderlich, das Reaktionsgemisch zu kühlen, da die Reaktionswärme durch das siedende Methylenchlorid abgeführt werden kann.

Bei Verwendung von höherkonzentrierter Salpetersäure kann es vorteilhaft sein, wenn man zu einer Suspension bzw. Lösung von Ausgangsprodukt und Methylenchlorid eine Mischung von Salpetersäure und Methylenchlorid zudosiert. In diesem Fall kann die Qualität an 3-Nitro-4-aminotoluol noch verbessert werden.

Es ist aber auch möglich, die Salpetersäure vorzulegen, gegebenenfalls im Gemisch mit Methylenchlorid, und das Einsatzprodukt zusammen mit dem Methylenchlorid als Lösung oder Suspension zuzugeben.

Bei der Durchführung der Nitrierung ist es günstig, wenn sich die Konzentration an Salpetersäure während der Reaktion so wenig wie möglich verändert. Man arbeitet daher bei Verwendung einer weniger als 98 bis 100 gew.-%igen Salpetersäure, z.B. so, dass man in die vorgelegte Suspension oder Lösung von p-Acetylaminotoluol und Methylenchlorid pro Mol p-Acetylaminotoluol zunächst die um 1 Mol verringerte Salpetersäuremenge der betreffenden Konzentration und anschliessend während der Reaktion noch 1 Mol 98 bis 100-%iger Salpetersäure zudosiert.

Die Aufarbeitung des Nitriergemisches kann in der Weise erfolgen, dass die Reaktionslösung in eine wässrige, etwa 1 bis 25 gew.-%ige*), bevorzugt 10 bis 20 gew.-%ige*) Lösung oder Suspension eines Ammonium-, Alkali- und/oder Erdalkalihydroxyds und/oder Carbonats, bevorzugt in eine wässrige Alkalyhydroxydlösung, besonders bevorzugt in wässrige Natronlauge, gegeben wird.

Dabei wird die Temperatur vorzugsweise so gewählt (z.B. 50 bis 70°C), dass Methylenchlorid zusammen mit Wasser abdestilliert.

Die anschliessende alkalische Entacetylierung wird bei etwa 80 bis 100°C, vorzugsweise bei 90 bis 95°C, durchgeführt.

Anschliessend wird bei etwa 0 bis 80°C, bevorzugt bei 50 bis 70°C, das ausgefallene Reaktions-

*) Die Angaben in Gew.-% beziehen sich auf den Gehalt an freier Base nach Neutralisation der überschüssigen Salpetersäure.

produkt abfiltriert und mit Wasser alkalifrei gewaschen.

Die Aufarbeitung des Nitriergemisches kann auch derart durchgeführt werden, dass das gesamte Reaktionsgemisch mit Wasser nahezu säurefrei gewaschen wird. Bevorzugt wird die überschüssige Salpetersäure und die in geringerer Menge enthaltenen Diazoniumsalze mit möglichst wenig Wasser diskontinuierlich oder kontinuierlich extrahiert. Auf diese Weise erhält man eine konzentrierte wässrige Salpetersäure-Lösung aus der Salpetersäure von etwa 55 bis etwa 68 Gew.-% sehr wirtschaftlich zurückgewonnen werden kann.

Das mit Wasser extrahierte Nitriergemisch wird, wie zuvor beschrieben, aufgearbeitet.

Nach erfolgter alkalischer Entacetylierung kann es auch vorteilhaft sein, das entacetylierte Produkt beispielsweise durch eine Flüssigphasentrennung, bevorzugt unter Druck, abzutrennen, oder in einem geeigneten, mit Wasser nicht mischbaren organischen Lösungsmittel, wie Methylenchlorid, Chlorbenzol, Dichlorbenzol oder Toluol entweder bei Raumtemperatur oder erhöhter Temperatur (30 bis 130°C) aufzunehmen, die organische Phase abzutrennen, mit Wasser alkalifrei zu waschen und das 3-Nitro-4-aminotoluol anschliessend z.B. durch Kristallisation, oder durch Destillation zu isolieren.

Die Ausbeuten an 3-Nitro-4-aminotoluol nach dem erfindungsgemässen Verfahren betragen je nach angewandter Säurekonzentration 92 bis 96% der Theorie; die Reinheiten liegen je nach Einsatzprodukt bei 98,4 bis 99,9%.

Falls es erforderlich sein sollte, kann das erhaltene 3-Nitro-4-aminotoluol noch weiter durch Umkristallisation, Sublimation oder Destillation gereinigt werden.

Gegenüber dem Verfahren nach dem Stand der Technik bietet das erfindungsgemässe Verfahren wesentliche Vorteile. So wird beim erfindungsgemässen Verfahren eine grössere Ausbeute und eine höhere Produktqualität (Reinheit) erzielt.

Weiterhin wird bei dem erfindungsgemässen Verfahren auf die Verwendung von Mineralsäuren als Lösungs- oder Verdünnungsmittel vollständig verzichtet. Dies bedingt gegenüber dem Stand der Technik nicht nur eine kostenmindernde Einsparung an Mineralsäure, sondern löst gleichzeitig die Probleme, die sonst mit der Aufarbeitung oder Beseitigung derartiger Säuremengen verbunden sind.

Ausserdem ist es ausserordentlich überraschend, dass bei dem erfindungsgemässen Verfahren keine Ablagerung von festen Diazoniumsalzen beobachtet werden konnte. Dies würde nämlich, wie in der DE-AS 2 226 405 ausdrücklich erwähnt, wegen der Gefährlichkeit der festen Diazoniumsalze eine technische Durchführung des Verfahrens ausschliessen.

3-Nitro-4-aminotoluol ist ein wichtiges Zwischenprodukt zur Herstellung von Farbstoffen (s. Ullmanns Enzyklopädie der technischen Chemie, 3. Auflage, Band 12, Seite 767).

Die nachfolgenden Beispiele sollen das erfindungsgemässe Verfahren erläutern, ohne es jedoch auf diese Beispiele einzuschränken.

Beispiel 1

In einem 2-l-Dreihalskolben, ausgerüstet mit Rührer, Rückflusskühler, Thermometer und Tropftrichter, wird eine Suspension von

298,4 g (2 Mol) 4-Acetylaminotoluol und
1200 ml Methylenchlorid

unter Normaldruck zum Sieden erhitzt und innerhalb von 30 Minuten werden

308,6 g 98%ige (4,8 Mol) Salpetersäure

zugetropft, wobei nach Zugabe von ca. der Hälfte der Säure eine Lösung entsteht. Nach beendetem Eintropfen der Salpetersäure wird noch 50 Minuten unter Rückfluss erhitzt und die erhaltene Lösung lässt man unter Rühren bei 50 bis 60°C in verdünnte Natronlauge, bestehend aus

680 ml Wasser und
232 g (5,8 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylamino-Gruppe zur Amino-Gruppe hydrolysiert. Es wird auf 60°C abgekühlt, ½ Stunde bei 60°C nachgerührt, abgesaugt und mit Wasser alkalifrei gewaschen.

Das Feuchtprodukt wird bis zur Gewichtskonstanz im Vakuum bei 60°C getrocknet.

Ausbeute: Produkt trocken 293,6 g (96,0% der Theorie).
Reinheit: 99,5%.

Beispiel 2

Der Versuch wurde wie im Beispiel 1 beschrieben, jedoch bei Einsatz von 298,4 g (2 Mol) mit Methylenchlorid gewaschenem technischem 4-Acetylaminotoluol durchgeführt.

Ausbeute: Produkt trocken 293,7 g (96,2% der Theorie).
Reinheit: 99,7%.

Beispiel 3

Der Versuch wurde wie im Beispiel 1 beschrieben, jedoch bei Einsatz von 304,5 g (2 Mol) 98 gew.-%igem technischem 4-Acetylaminotoluol, durchgeführt.

Beispiel 4

In einem 2-l-Dreihalskolben, ausgerüstet mit Rührer, Rückflusskühler, Thermometer und Tropftrichtern wird eine Suspension von

304,5 g (2 Mol) 98 %igem technischem 4-Acetylaminotoluol und
1200 ml Methylenchlorid

unter Normaldruck zum Sieden erhitzt.

Durch den Rückflusskühler werden innerhalb von 30 Minuten

308,6 g 98%ige (4,8 Mol) Salpetersäure

zugetropft, wobei nach Zugabe von ca. der Hälfte der Säure eine Lösung entsteht. Nach beendetem Eintropfen der Salpetersäure wird noch 50 Minuten unter Rückfluss erhitzt und die erhaltene Lösung lässt man unter Rühren bei 50 bis 60°C in verdünnte Natronlauge, bestehend aus

589,8 ml Wasser und
216 g (5,4 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylaminogruppe zur Aminogruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 294,9 g (95,7% der Theorie).
Reinheit: 98,8%.

Beispiel 5

In einem 2 l Dreihalskolben, ausgerüstet mit Rührer, Rückflusskühler, Thermometer und Tropftrichter, wird eine Suspension von

304,5 g (2 Mol) 98 %igem technischem 4-Acetylaminotoluol und
1200 ml Methylenchlorid

unter Normaldruck zum Sieden erhitzt und innerhalb von 30 Minuten werden

308,6 g 98 %ige (4,8 Mol) Salpetersäure

zugetropft, wobei nach Zugabe von ca. der Hälfte der Säure eine Lösung entsteht. Nach beendetem Eintropfen der Salpetersäure wird noch 50 Minuten unter Rückfluss erhitzt und die erhaltene Lösung lässt man unter Rühren bei 50 bis 60°C in verdünnte Natronlauge, bestehend aus

589,8 ml Wasser und
216 g (5,4 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylaminogruppe zur Aminogruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 295,2 g (95,5% der Theorie).
Reinheit: 98,4%.

Beispiel 6
In einem 2-l-Dreihalskolben, ausgerüstet mit Rührer, Rückflusskühler, Thermometer und Tropftrichter, wird eine Suspension von

304,5 g (2 Mol) 98%igem technischem 4-Acetylaminotoluol und
1200 ml Methylenchlorid

unter Normaldruck zum Sieden erhitzt.
Durch den Rückflusskühler werden innerhalb von 30 Minuten

308,6 g 98%ige (4,8 Mol) Salpetersäure

zugetropft, wobei nach Zugabe von ca. der Hälfte der Säure eine Lösung entsteht. Nach beendetem Eintropfen· der Salpetersäure wird noch 50 Minuten unter Rückfluss erhitzt und die erhaltene Lösung lässt man unter Rühren bei 50 bis 60°C in verdünnte Natronlauge, bestehend aus

680 ml Wasser und
232 g (5,8 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylaminogruppe zur Aminogruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 294,1 g (95,5% der Theorie).
Reinheit: 98,8%.

Beispiel 7
Der Versuch wurde wie im Beispiel 6 beschrieben, jedoch bei Einsatz von

a) 900 ml Methylenchlorid,
b) 4000 ml Methylenchlorid,

durchgeführt.

a) Ausbeute: Produkt trocken 292,8 g (94,9% der Theorie)
Reinheit: 98,6%

b) Ausbeute: Produkt trocken 295,0 g (95,9% der Theorie)
Reinheit: 98,9%

Beispiel 8
In einem 2 l Dreihalskolben, ausgerüstet mit Rührer, Rückflusskühler, Thermometer und Tropftrichter, wird eine Suspension von

304,5 g (2 Mol) 98%igem technischem 4-Acetylaminotoluol und
1200 ml Methylenchlorid

unter Normaldruck zum Sieden erhitzt und innerhalb von 30 Minuten wird eine Mischung von

308,6 g 98%ige (4,8 Mol) Salpetersäure und
308 g Methylenchlorid

zugetropft, wobei nach Zugabe von ca. der Hälfte der Säure eine Lösung entsteht. Nach beendetem Eintropfen des Salpetersäure-Methylenchlorid-Gemisches wird noch 50 Minuten unter Rückfluss erhitzt und die erhaltene Lösung lässt man unter Rühren bei 50 bis 60°C in verdünnte Natronlauge, bestehend aus

589,8 ml Wasser und
216 g (5,4 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylaminogruppe zur Aminogruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 295,3 g (95,8% der Theorie).
Reinheit: 98,7%.

Beispiel 9
In einem 3 l-Dreihalskolben, ausgerüstet mit Rührern, Rückflusskühler, Thermometer und Tropftrichter, wird eine Suspension von

298.4 g (2 Mol) 4-Acetylaminotoluol und
1200 ml Methylenchlorid

unter Nordmaldruck zum Sieden erhitzt und innerhalb von 30 Minuten werden zunächst

570 g 66,3%ige (6 Mol) Salpetersäure

und innerhalb weiterer 30 Minuten

128,6 g 98%ige (2 Mol) Salpetersäure

zugetropft, wobei schon während der Zugabe der Salpetersäure eine Lösung entsteht. Nach beendetem Eintropfen der Salpetersäure wird noch 30 Minuten unter Rückfluss erhitzt und die erhaltene Lösung lässt man unter Rühren bei 50 bis 60°C in verdünnte Natronlauge, bestehend aus

680 ml Wasser und
360 g (9 Mol) NaOH

einfliessen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylaminogruppe zur Amino-Gruppe hydrolyisert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 284,1 g (92,4% der Theorie).
Reinheit: 99,0%.

Beispiel 10

In einem 2 l-Dreihalskolben, ausgerüstet mit Rührer, Rückflusskühler, Thermometer und Tropftrichter, wird eine Suspension von

298,4 g (2 Mol) 4-Acetylaminotoluol und
1200 ml Methylenchlorid

unter Normaldruck zum Sieden erhitzt und innerhalb von 30 Minuten werden

308,6 g 98%ige (4,8 Mol) Salpetersäure

zugetropft, wobei nach Zugabe von ca. der Hälfte der Säure eine Lösung entsteht. Nach beendetem Eintropfen der Salpetersäure wird noch 50 Minuten unter Rückfluss erhitzt, die erhaltene Lösung mit 100 ml Wasser versetzt, der Rührer abgestellt und die beiden Phasen abgetrennt. Die untere Methylenchloridphase wird noch 3 mal mit je 75 ml Wasser extrahiert, dann unter Rühren bei 50-60°C in verdünnte Natronlauge, bestehend aus

680 ml Wasser und
120 g (3 Mol) NaOH

einfliessen gelassen, wobei Methylenchlorid mit 25 bis 30 ml Wasser abdestilliert. Nachdem die gesamte Menge an Methylenchlorid abdestilliert ist, wird die Temperatur bis auf 95°C gesteigert und 1½ Stunden gehalten, wobei die Acetylamino-Gruppe zur Amino-Gruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 288,7 g (94,8% der Theorie)
Reinheit: 99,9%

Die vereinigten wässrigen Phasen des Nitriergemisches, bei denen es sich um etwa 540 g einer etwa 30 bis 33-%igen Salpetersäure handelt, werden bis zum Sieden erhitzt und durch Abdestillation von Wasser und Salpetersäure auf eine etwa 55%ige Salpetersäure, anschliessend durch Destillation mit konzentrierter Schwefelsäure auf eine ca. 98%ige Salpetersäure aufkonzentriert und bei den nächsten Versuchen wieder eingesetzt.

Beispiel 11

Der Versuch wurde wie im Beispiel 10 beschrieben, jedoch bei Einsatz von 298,4 g (2 Mol) mit Methylenchlorid gewaschenem technischem 4-Acetylaminotoluol durchgeführt.

Ausbeute: Produkt trocken 298,8 g (95,0% der Theorie)
Reinheit: 99,8%.

Beispiel 12

Der Versuch wurde wie im Beispiel 10 beschrieben, jedoch bei Einsatz von 304,5 g (2 Mol) 98 gew.-%igem technischem 4-Acetylaminotoluol durchgeführt.

Ausbeute: Produkt trocken 287,6 g (93,6% der Theorie)
Reinheit: 99,0%.

Beispiel 13

Es wurde wie im Beispiel 12 beschrieben, jedoch wurde die Salpetersäure durch den Rückflusskühler zudosiert.

Ausbeute: Produkt trocken 287,9 g (93,8% der Theorie)
Reinheit: 99,1%.

Beispiel 14

Es wurde wie in den Beispielen 10, 11, 12 und 13 verfahren, jedoch wurde die extrahierte Methylenchlorid-Phase in verdünnte Natronlauge, bestehend aus

498 ml Wasser und
88 g (2 ,2 Mol) NaOH

einfliessen gelassen.

Die Ausbeute und Reinheit der Produkte war praktisch die gleiche, wie bei den entsprechenden Beispielen 10, 11, 12 und 13.

Beispiel 15

In einem 3 l-Dreihalskolben, ausgerüstet mit Rührer, Rückflusskühler, Thermometer und Tropftrichter, wird eine Suspension von

298,4 g (2 Mol) 4-Acetylamonotoluol und
1200 ml Methylenchlorid

unter Normaldruck zum Sieden erhitzt und innerhalb von 30 Minuten werden zunächst

570 g 66,3%ige (6 Mol) Salpetersäure

und innerhalb weiterer 30 Minuten

128,6 g 98%ige (2 Mol) Salpetersäure

zugetropft, wobei schon während der Zugabe der Salpetersäure eine Lösung entsteht. Nach beendetem Eintropfen der Salpetersäure wird noch 30 Minuten unter Rückfluss erhitzt und anschliessend wie in Beispiel 10 beschrieben, weiterverfahren.

Ausbeute: Produkt trocken 279,6 g (91,8% der Theorie)
Reinheit: 99,8%.

Beispiel 16 (Nacharbeitung des Beispiels 4 der US-PS 2 459 002)

Der Versuch wurde in einem 1 Mol Ansatz wie im Beispiel 4 der US-PS beschrieben als Eintopfverfahren ohne Zwischenisolierung des 3-Nitro-4-acetylaminotoluols mit anschliessender saurer Spaltung durchgeführt.
Das Feuchtprodukt wurde bis zur Gewichtskonstanz im Vakuum bei 60°C getrocknet.

Ausbeute: Produkt trocken 89,0 g (50,5% der Theorie)
Reinheit: 86,4%.

Beispiel 17 (Vergleichsbeispiel)
Es wurde wie im Beispiel 16 verfahren, jedoch wurde die heisse, wässrige und neutrale Suspension nach der Wasserdampfdestillation mit 60 g (1,5 Mol) NaOH versetzt, auf 95°C erhitzt und 1½ Stunden bei dieser Temperatur gehalten, wobei die Acetylamino-Gruppe zur Amino-Gruppe hydrolysiert. Es wird auf 60°C abgekühlt, ½ Stunde bei 60°C nachgerührt, abgesaugt und mit Wasser alkalifrei gewaschen. Das Feuchtprodukt wird bis zur Gewichtskonstanz im Vakuum bei 60°C getrocknet.

Ausbeute: Produkt trocken 134,5 g (82,4% der Theorie)
Reinheit: 93,2%.

**Patentansprüche**

1. Verfahren zur Herstellung von 3-Nitro-4-aminotoluol durch Nitrieren von p-Acetylaminotoluol in Gegenwart von inerten, mit Wasser nicht mischbaren organischen Lösungsmitteln bei Temperaturen im Bereich von 0 bis 80°C und anschliessender alkalischer Verseifung und Aufarbeitung, dadurch gekennzeichnet, dass man die Nitrierung mit 60- bis 100-gew.-%iger Salpetersäure in Gegenwart von Methylenchlorid durchführt.

2. Verfahren nach Anspruch 1, dadurch gekennzeichnet, dass man pro Mol p-Acetylaminotoluol 2 bis 6 Mol 100-gew.-%ige Salpetersäure einsetzt.

3. Verfahren nach Anspruch 1, dadurch gekennzeichnet, dass man pro Mol p-Acetylaminotoluol 2,2 bis 4,5 Mol 100-gew.-%ige Salpetersäure einsetzt.

4. Verfahren nach Anspruch 1, dadurch gekennzeichnet, dass man pro Mol p-Acetylaminotoluol 6 bis 12 Mol 60-gew.-%ige Salpetersäure einsetzt.

5. Verfahren nach Anspruch 1, dadurch gekennzeichnet, dass man pro Mol p-Acetylaminotoluol 6,5 bis 8 Mol 60-gew.-%ige Salpetersäure einsetzt.

6. Verfahren nach Ansprüchen 1 bis 5, dadurch gekennzeichnet, dass man pro Mol p-Acetylaminotoluol 400 bis 2000 ml Methylenchlorid einsetzt.

7. Verfahren nach Ansprüchen 1 bis 6, dadurch gekennzeichnet, dass man pro Mol p-Acetylaminotoluol 500 bis 700 ml Methylenchlorid einsetzt.

**Revendications**

1. Procédé de fabrication de 3-nitro-4-aminotoluène par nitration du p-acétylamino-toluène en présence de solvants organiques inertes non miscibles avec l'eau, à des températures dans l'intervalle de 0 à 80°C et avec saponification alcaline consécutive puis traitement, caractérisé en ce qu'on exécute la nitration avec de l'acide nitrique à 60–100% en poids en présence de chlorure de méthylène.

2. Procédé selon la revendication 1, caractérisé en ce que par mole de p-acétylamino-toluène on utilise 2 à 6 moles d'acide nitrique à 100% en poids.

3. Procédé selon la revendication 1, caractérisé en ce que par mole de p-acétylaminotoluène on utilise 2,2 à 4,5 moles d'acide nitrique à 100% en poids.

4. Procédé selon la revendication 1, caractérisé en ce que par mole de p-acétylaminotoluène on utilise 6 à 12 moles d'acide nitrique à 60% en poids.

5. Procédé selon la revendication 1, caractérisé en ce que par mole de p-acétylaminotoluène on utilise 6,5 à 8 moles d'acide nitrique à 60% en poids.

6. Procédé selon les revendications 1 à 5, caractérisé en ce que par mole de p-acétylaminotoluène on utilise 400 à 2000 ml de chlorure de méthylène.

7. Procédé selon les revendications 1 à 6, caractérisé en ce que par mole de p-acétylaminotoluène on utilise 500 à 700 ml de chlorure de méthylène.

**Claims**

1. Process for the preparation of 3-nitro-4-aminotoluene by nitration of p-acetylaminotoluene in the presence of inert, water-immiscible organic solvents at temperatures in the range from 0 to 80°C and by subsequent alkaline saponification and working up, characterised in that the nitration is carried out with 60 to 100% strength by weight nitric acid in the presence of methylene chloride.

2. Process according to Claim 1, characterised in that 2 to 6 mols of 100% strength by weight nitric acid are employed per mol of p-acetylaminotoluene.

3. Process according to Claim 1, characterised in that 2.2 to 4.5 mols of 100% strength by weight nitric acid are employed per mol of p-acetylaminotoluene.

4. Process according to Claim 1, characterised in that 6 to 12 mols of 60% strength by weight nitric acid are employed per mol of p-acetylaminotoluene.

5. Process according to Claim 1, characterised in that 6.5 to 8 mols of 60% strength by weight nitric acid are employed per mol of p-acetylaminotoluene.

6. Process according to Claims 1 to 5, characterised in that 400 to 2,000 ml of methylene chloride are employed per mol of p-acetylaminotoluene.

7. Process according to Claims 1 to 6, characterised in that 500 to 700 ml of methylene chloride are employed per mol of p-acetylaminotoluene.